# EUROPÄISCHE PATENTSCHRIFT

(11) **EP 1 740 528 B1**
(45) Veröffentlichungstag und Bekanntmachung des Hinweises auf die Patenterteilung: **08.10.2008**
(21) Anmeldenummer: 05732739.7
(22) Anmeldetag: 20.04.2005
(51) Int. Cl.: C07C 67/08

(54) **EMULGATORKONZENTRAT FÜR KOSMETISCHE ZUSAMMENSETZUNG**
EMULSIFIER CONCENTRATE FOR A COSMETIC COMPOSITION
CONCENTRE D'AGENT EMULSIFIANT POUR COMPOSITION COSMETIQUE

(30) Priorität: 29.04.2004 DE 102004021312
(43) Veröffentlichungstag der Anmeldung: 10.01.2007
(73) Patentinhaber: Cognis IP Management GmbH, 40589 Düsseldorf (DE)
(72) Erfinder: NEUSS, Michael, 50997 Köln (DE); ALBERS, Thomas, 40597 Düsseldorf (DE)
(86) Internationale Anmeldenummer: PCT/EP2005/004197
(87) Internationale Veröffentlichungsnummer: WO 2005/107681

(56) Entgegenhaltungen:
- EP-A- 0 000 424
- WO-A-98/55088
- WO-A-20/04041769
- WO-A-20/05016301
- US-A1- 2004 248 748

## Beschreibung

### Gebiet der Erfindung

Die Erfindung betrifft bei 20°C pumpbare Emulgator-Konzentrate, deren Verwendung sowie ein Verfahren zur Herstellung von Emulsionen unter Verwendung des Emulgator-Konzentrates.

### Stand der Technik

Die Herstellung stabiler, emulsionsförmiger Zubereitungen erfordert normalerweise einen erheblichen apparativen Aufwand, da die Emulgierung von Öl- und Wasserphase meist bei erhöhten Temperaturen vorgenommen werden muss. Zu den festen oder wachsartigen Komponenten der Ölphase, die aufgeschmolzen werden müssen, gehören u.a. eine Vielzahl der W/O-Emulgatoren. Eine Dosierung dieser festen Stoffe in großtechnischen Prozessen ist wesentlich aufwendiger als die von flüssigen Komponenten. Zu den wachsartigen, in Kosmetika sehr häufig eingesetzten W/O-Emulgatoren zählen die Polyethylenglycol-Diester (PEG-Diester). Von diesen ist eine Vielzahl bei Raumtemperatur in den üblichen, in der Kosmetik verwendeten Ölkomponenten allerdings unlöslich. Selbst bei einem Verhältnis von 10 Gew.-% PEG-Diester und 90 Gew.-% Ölkomponenten bildet sich ein Bodensatz bzw. pastöse oder wachsartige Produkte.

Es bestand die Aufgabe, PEG-Diester-Konzentrate zur Verfügung zu stellen, die bei 20 °C fließfähig und pumpbar sind und sich leicht in emulsionsförmige Zubereitungen einarbeiten lassen. Der Gehalt des PEG-Diesters im Konzentrat sollte dabei möglichst hoch und das Konzentrat auch kalt verarbeitbar sein.

### Beschreibung der Erfindung

Gegenstand der Erfindung ist ein bei 20°C fließfähiges Emulgator-Konzentrat enthaltend
(a) bis zu 90 Gew.-% eines Polyethylenglycol-Fettsäure-Diesters auf Basis einer C16-C22-Fettsäure, einer C16-C22-Hydroxyfettsäure, Polyhydroxystearinsäure oder Poly-(C16-C22-Hydroxyfettsäure)
(b) bis zu 80 Gew.-% eines bei 25°C flüssigen Ölkörpers oder Ölkörpergemisches und
(c) 2 - 8 Gew.-% Wasser.

Vorzugsweise enthalten die Konzentrate bis auf rohstoffbedingte Verunreinigungen keine weiteren Bestandteile, so dass sie im Wesentlichen aus den Komponenten (a) bis (c) bestehen. Die Komponente (a) ist vorzugsweise in einer Menge von bis zu 85 Gew.% im Konzentrat enthalten. In einer bevorzugten Ausführungsform enthält das Emulgator-Konzentrat (a) 10 - 80 Gew.-% eines Polyethylenglycol-Fettsäure-Diesters auf Basis einer C16-C22-Fettsäure, einer C16-C22-Hydroxyfettsäure, Polyhydroxystearinsäure oder Poly(C16-C22-Hydroxyfettsäure), (b) 10- 80 Gew.-% eines bei 25°C flüssigen Ölkörpers oder Ölkörpergemisches und (c) 2 - 8 Gew.-% Wasser. Besonders bevorzugt sind Konzentrate mit (a) 30 - 60 Gew.-% eines Polyethylenglycol-Fettsäure-Diesters auf Basis einer C16-C22-Fettsäure, einer C16-C22-Hydroxyfettsäure, Polyhydroxy-stearinsäure oder Poly(C16-C22-Hydroxyfettsäure), (b) 30 - 60 Gew.-% eines bei 25°C flüssigen Ölkörpers oder Ölkörpergemisches und (c) 3 - 6 Gew.-% Wasser. Ganz besonders bevorzugt ist eine Konzentrat-Zusammensetzung von (a) 35 - 55 Gew.-% eines Polyethylenglycol-Fettsäure-Diesters auf Basis einer C16-C22-Fettsäure, einer C16-C22-Hydroxyfettsäure, Polyhydroxy-stearinsäure oder Poly(C16-C22-Hydroxyfettsäure), (b) 40 - 60 Gew.-% eines bei 25°C flüssigen Ölkörpers oder Ölkörpergemisches und (c) 3 - 6 Gew.-% Wasser.

Überraschenderweise wurde gefunden, dass sich die Polyethylenglycol-Fettsäure-Diester (a) in flüssigen Ölkörpern lösen, wenn eine definierte Menge an Wasser zugesetzt wird. Die Wassermenge ist hierbei ein kritischer Parameter. Emulgator-Konzentrate, die Mengen bis zu 90 Gew.-% eines Polyethylenglycol-Fettsäure-Diesters enthalten, sind in Ölkörpern unterschiedlichster Polarität löslich, wenn 2 - 8 Gew.-% Wasser in den Konzentraten enthalten ist. Bei einem Wassergehalt unterhalb von 2 Gew.-% bildet sich ein Bodensatz bzw. die Mischung wird pastös bis fest, bei Mengen oberhalb von 8 Gew.-% kommt es zur Vergelung und die Konzentrate sind nicht mehr pump- oder fließfähig. Bevorzugt sind Wassermengen von 3 - 6 Gew.-%, und besonders bevorzugt sind Wassermengen von 4 - 5 Gew.-% bezogen auf die Gesamtmenge des Konzentrates.

Eine bevorzugte Ausführungsform des Emulgator-Konzentrates ist dadurch gekennzeichnet, dass es keine weiteren nichtionischen, anionischen, kationischen amphoteren und/oder zwitterionischen oberflächenaktiven Substanzen enthält. Insbesondere enthält das erfindungsgemäße Konzentrat keine UV-Filter und/oder Pigmente. Vorzugsweise besteht das Konzentrat im Wesentlichen aus den Komponenten (a), (b) und (c), wobei der Polyethylenglycol-Diester herstellungsbedingt auch Rest-Anteile an Monoester, unveresterter Fettsäure, Polyethylenglycol und Polyethylenglycol-Monoester enthalten kann. Die Polyethlyenglycol-Diester selbst sind seit langem bekannt und werden von zahlreichen Firmen auf dem Markt angeboten. Die PEG-Einheit der Polyethylenglycol-Diester der erfindungsgemäßen Konzentrate weist ein Molekulargewicht von 600 - 3.000 auf, vorzugsweise von 1.000 - 2.000, besonders bevorzugt von ca. 1.500. Dies entspricht im Mittel 30 repititiven Oxyethylen-Einheiten.

Besonders geeignet sind Emulgator-Konzentrate, die als Komponente a) einen Ester enhalten, der dadurch erhältlich ist, dass man eine Fettsäure und/oder Hydroxyfettsäure mit 16 bis 22 C-Atomen oder eine entsprechende Polyfettsäure und/oder Polyhydroxyfettsäure mit einem Eigenkondensationsgrad von 2 bis 20, insbesondere 2 bis 10, mit einem Polyethylenglykol unter Durchführung einer Veresterungsreaktion in Gegenwart eines Katalysators umsetzt, der eine anorganische Phosphor(I)-Verbindung sowie ein Titanat umfasst, wobei die Carbonylverbindung und die anorganische Phosphor(I)-Verbindung miteinander vermischt werden, die erhaltene Mischung filtriert wird und anschließend der filtrierten Mischung der Alkohol und das Titanat zugesetzt werden und die Veresterungsreaktion durchgeführt wird. Ein derartiges Verfahren, das besonders reine und farblose PEG-Diester liefert, ist Gegenstand der DE 102 51 984**.**

Im Sinne der Erfindung sind Emulgator-Konzentrate bevorzugt, die bei 20°C eine Viskosität von weniger als 20.000 mPa·s aufweisen (Brookfield Viskosimeter, Spindel 5, 10 Umdrehungen pro Minute). Erfindungsgemäß besonders bevorzugt sind Emulgator-Konzentrate, die bei 15°C eine Viskosität von weniger als 10.000 mPa·s aufweisen. In einer weiteren bevorzugten Ausführungsform sind die Emulgator-Konzentrate transparent oder transluzent.

### Ölkörper

Als Ölkörper für das Emulsionskonzentrat eignen sich alle in der Kosmetik üblicherweise verwendeten Öle oder deren Gemische, die bei 25 °C unter Normaldruck flüssig sind. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen. Beispielhaft seien genannt Hexyllaurat, Myristylisostearat, Myristyloleat, Cetylisostearat, Cetyloleat, Stearylisostearat, Stearyloleat, Isostearylmyristat, Isostearylpalmitat, Isostearylstearat, Isostearylisostearat, Isostearyloleat, Oleylmyristat, Oleylisostearat, Oleyloleat, Oleylerucat, Erucylisostearat, Erucyloleat, Cococaprylat/caprat. Daneben eignen sich Ester von linearen C₆-C₂₂-Fettsäuren mit verzweigten Alkoholen, insbesondere 2-Ethylhexanol und Isopropanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, flüssige Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylylcarbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv^{®} TN), lineare oder verzweigte, symmetrische oder unsymmetrische Dialkylether mit 6 bis 22 Kohlenstoffatomen pro Alkylgruppe, wie z. B. Dicaprylyl Ether (Cetiol^{®} OE), Ringöffnungsprodukte von epoxidierten Fettsäureestern mit Polyolen, Abmischungen dieser Ölkörper mit Silikonölen (Cyclomethico ne, Siliciummethicontypen u.a.) und/oder aliphatische bzw. naphthenische Kohlenwasserstoffe, wie z. B. Mineralöl, Vaseline, Petrolatum, Isohexadecane, Squalan, Squalen oder Dialkylcyclohexane in Betracht. Erfindungsgemäß bevorzugt sind Emulgator-Konzentrate, die Ölkörper enthalten, die ausgewählt sind aus der Gruppe der Dialkylether, der Dialkylcarbonate, der Triglyceride, der Ester, der Kohlenwasserstoffe, der verzweigten C12-C24-Fettalkohole oder einem Gemisch dieser Substanzen. Besonders bevorzugt ist es, dass der Ölkörper im wesentlichen aus Dialkylcarbonaten oder Dialkylethern oder einem Gemisch dieser Komponenten besteht, dass also andere Ölkörper lediglich herstellungsbedingt als Verunreinigungen enthalten sind. In einer besonders bevorzugten Ausführungsform ist der Ölkörper Dioctylether oder Dioctylcarbonat oder ein Gemisch dieser beiden Substanzen.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Eine weitere bevorzugte Ausführungsform des Emulgator-Konzentrates ist dadurch gekennzeichnet, dass es ein Hydrotrop ausgewählt aus der Gruppe der Polyole 2 bis 15 Kohlenstoffatomen und mindestens 2 Hydroxylgruppen enthält. Diese verbessern auch das Kältefließverhalten des Konzentrates.

### Kosmetische Zubereitungen

Die erfindungsgemäßen Emulgator-Konzentrate erlauben die Herstellung von Emulsionen in einem Kaltprozeß, wenn alle weiteren Bestandteile flüssig sind. Ein weiterer Gegenstand der Erfindung ist die Verwendung der erfindungsgemäßen Emulgator-Konzentrate in kosmetischen und pharmazeutischen Emulsionen. Gegenstand ist auch ein Verfahren zur Herstellung von Emulsionen, das dadurch gekennzeichnet ist, dass man das erfindungsgemäße Emulgator-Konzentrat mit einer Öl- und einer Wasserphase in einem Heiß- oder Kaltprozeß emulgiert, welche die üblichen Hilfs- und Zusatzstoffe sowie weitere Emulgatoren enthalten können. Vorzugsweise handelt es sich hierbei um Formulierungen zur Körperpflege, z. B. in Form von Cremes, Milchen, Lotionen, sprühbaren Emulsionen, Produkten zur Eliminierung des Körpergeruchs etc. Die erfindungsgemäße Verbindung lässt sich auch in tensidhaltigen Formulierungen wie z. B. Schaum- und Duschbädem, Haarshampoos und Pflegespülungen einsetzen.

Die kosmetischen Mittel können in Form von Emulsionen oder Dispersionen vorliegen. Bevorzugte kosmetische Zusammensetzungen sind solche in Form einer W/O- oder O/W-Emulsion mit den üblichen, dem Fachmann geläufigen, Konzentrationen an Ölen/ Fetten/Wachsen, Emulgatoren, Wasser und den in der Kosmetik üblichen, weiteren Hilfs- und Zusatzstoffen.

Zweckmäßig enthalten die erfindungsgemäßen kosmetische Zusammensetzungen 1 bis 30 Gew.-%, bevorzugt 3 bis 20 Gew.-% und insbesondere 5 bis 15 Gew.-% des erfindungsgemäßen Emulgator-Konzentrates.

Je nach Applikationszweck enthalten die kosmetischen Formulierungen eine Reihe weiterer Hilfs- und Zusatzstoffe wie beispielsweise oberflächenaktive Substanzen (Tenside, Emulgatoren), weitere Ölkörper, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungs-mittel), Hydrotrope, Solubilisatoren, Konservierungsmittel, Parfümöle, Farbstoffe etc., von denen einige nachstehend exemplarisch aufgelistet sind.

Die Mengen der jeweiligen Zusätze richten sich nach der beabsichtigten Verwendung.

### Oberflächenaktive Substanzen

Als oberflächenaktive Stoffe können anionische, nichtionische, kationische und/oder amphotere bzw. zwitterionische Tenside bzw. Emulgatoren oder ein beliebiges Gemisch dieser Tenside/Emulgatoren enthalten sein. In tensidhaltigen kosmetischen Zubereitungen, wie beispielsweise Duschgelen, Schaumbädern, Shampoos etc. ist vorzugsweise wenigstens ein anionisches Tensid enthalten, in Cremes und Lotionen für die Körperpflege sind vorzugsweise weitere nicht-ionische Tenside/Emulgatoren enthalten.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside/Emulgatoren** sind Fettalkoholpolyglycolether, Polyglycerinester, Alkylphenolpolyglycolether, Fettsäurepolyglycolmonoester, Fettsäureamidpolyglycolether, Fettaminpolyglycolether, alkoxylierte Triglyceride, Mischether bzw. Mischformale, Alk(en)yloligoglykoside bzw. Glucoronsäurederivate - gegebenenfalls partiell oxidiert, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Körperpflegemittel wie Cremes und Lotionen enthalten üblicherweise eine Reihe weiterer Ölkörper und Emollients, die dazu beitragen, die sensorischen Eigenschaften weiter zu optimieren. In die kosmetischen Endformulierungen können alle für kosmetische Applikationen geeigneten Ölkörper eingearbeitet werden. Hierfür eignen sich alle Ölkörper, die beispielhaft als Ölkörper für das Emulgator-Konzentrat genannt wurden (*vide supra*).

Die kosmetischen Zusammensetzungen können ferner auch weitere Hydrotrope enthalten, die bereits für das Konzentrat genannt wurden.

### Fette und Wachse

Fette und Wachse werden den Körperpflegeprodukten als Pflegestoffe zugesetzt und auch, um die Konsistenz der Kosmetika zu erhöhen. Typische Beispiele für Fette sind Glyceride, d. h. feste oder flüssige pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage.

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Verdickungsmittel

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyacrylate, (z. B. Carbopole^{®} und Pemulen-Typen von Goodrich; Synthalene^{®} von Sigma; Keltrol-Typen von Kelco; Sepigel-Typen von Seppic; Salcare-Typen von Allied Colloids und Cosmedia^{®} SP und SPL von Cognis), Polyacrylamide, Polymere, Polyvinylalkohol, Polyvinylpyrrolidon und Bentonite wie z. B. Bentone^{®} Gel VS-5PC (Rheox). Weiter in Frage kommen Elektrolyte wie Kochsalz und Ammoniumchlorid.

### Stabilisatoren

Als Stabilisatoren können Metallsalze von Fettsäuren, wie z. B. Magnesium-, Aluminium- und/oder Zinkstearat bzw. -ricinoleat eingesetzt werden.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UV-B-Filter können öllöslich oder wasserlöslich sein. Als typische UV-A-Filter kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z.B. 4-tert.-Butyl-4'-methoxydibenzoylmethan (Parsol^{®} 1789) und 2-Cyano-3,3-phenylzimtsäure-2-ethyl-hexylester (Octocrylene) sowie Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Häufig werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen auch unlösliche Lichtschutzpigmente, nämlich feindisperse Metalloxide bzw. Salze in Frage. Beispiele für geeignete Metalloxide sind insbesondere Zinkoxid und Titandioxid. Als Salze können Silicate (Talk), Bariumsulfat oder Zinkstearat eingesetzt werden. Die Oxide und Salze werden in Form der Pigmente für hautpflegende und hautschützende Emulsionen verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

### Desodorierende Wirkstoffe

Desodorierende Wirkstoffe wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

### Antitranspirante Wirkstoffe

Antitranspirant-Wirkstoffe reduzieren durch Beeinflussung der Aktivität der ekkrinen Schweißdrüsen die Schweißbildung, und wirken somit Achselnässe und Körpergeruch entgegen. Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2, Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin.

### Insekten-Repellentien

Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

### Selbstbräuner und Depigmentierungsmittel

Als Selbstbräuner eignet sich Dihydroxyaceton. Als Tyrosinhinbitoren, die die Bildung von Melanin verhindem und Anwendung in Depigmentierungsmitteln finden, kommen beispielsweise Arbutin, Ferulasäure, Kojisäure, Cumarinsäure und-Ascorbinsäure (Vitamin C) in Frage.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

### Farbstoffe

Als Farbstoffe können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Diese Farbstoffe werden üblicherweise in Konzentrationen von 0,001 bis 0,1 Gew.-%, bezogen auf die gesamte Mischung, eingesetzt.

### Beispiele

Die Erfindung soll nachfolgend anhand von Beispielen weiter erläutert werden.

### 1. Synthese der Polyethylenglycol-Fettsäure-Diester

### Beispiel A

### Synthese eines Polyethylenglycol-1500-polyhydroxystearats

726,8 g (2,37 mol) 12-Hydroxystearinsäure, 273,2 g (0,189 mol) Polyethylenglycol-1500 und 0,2 g Tetrabutyltitanat werden unter Stickstoff stufenweise auf 240°C aufgeheizt. Nach Beendigung der Wasserabscheidung wird Vakuum angelegt und weiter kondensiert, bis keine weitere Absenkung der Säurezahl zu beobachten ist. Nach Abkühlung auf 100°C und Zugabe von 0,5% Filterhilfsmittel (Hyflow^{®} Supercel) wird das Produkt filtriert.
Das Produkt (Filtrat) besitzt eine Säurezahl von 9,5. Die Farbe ist dunkelbraun, die Farbzahl nach Hazen bzw. Gardner ist nicht bestimmbar. Die Konsistenz ist (bei 20 °C) wachsartig-fest.

### Beispiel B

### Synthese eines Polyethylenglycol-1000-polyhydroxystearats

754,3 g (2,46 mol) 12-Hydroxystearinsäure, 245,8 g (0,246 mol) Polyethylenglycol-1000 und 0,1 g Zinnoxid werden unter Stickstoff stufenweise auf 240°C aufgeheizt. Nach Beendigung der Wasserabscheidung wird Vakuum angelegt und weiter kondensiert, bis keine weitere Absenkung der Säurezahl zu beobachten ist. Nach Abkühlung auf 100°C und Zugabe von 0,5% Filterhilfsmittel (Hyflow^{®} Supercel) wird das Produkt filtriert.
Das Produkt (Filtrat) besitzt eine Säurezahl von 17. Die Farbe ist dunkelbraun, die Farbzahl nach Hazen bzw. Gardner ist nicht bestimmbar. Die Konsistenz ist (bei 20 °C) wachsartig-fest.

### Beispiel C

### Synthese eines Polyethylenglycol-3000-polyhydroxystearats

506 g (1,65 mol) 12-Hydroxystearinsäure, 494 g (0,165 mol) Polyethylenglycol-3000 und 0,1 g Zinnoxid werden unter Stickstoff stufenweise auf 240°C aufgeheizt. Nach Beendigung der Wasserabscheidung wird Vakuum angelegt und weiter kondensiert, bis keine weitere Absenkung der Säurezahl zu beobachten ist. Nach Abkühlung auf 100°C und Zugabe von 0,5% Filterhilfsmittel (Hyflow^{®} Supercel) wird das Produkt filtriert.

Das Produkt (Filtrat) besitzt eine Säurezahl von 18. Die Farbe ist dunkelbraun, die Farbzahl nach Hazen bzw. Gardner ist nicht bestimmbar. Die Konsistenz ist (bei 20 °C) wachsartig-fest.

### Beispiel D

### Synthese eines Polyethylenglycol-1500-polyhydroxystearats

726,8 g (2,37 Mol) 12-Hydroxystearinsäure werden mit 5 g Phosphor(I)-säure (50 %ig) versetzt und 1 Stunde lang bei 90°C gerührt. Nach Zugabe von 8 g Natriumcarbonat und 5 g Hyflow^{®} Supercel wird die heiße Mischung filtriert. Das Filtrat wird mit 273,2 g (0,189 Mol) Polyethylenglycol-1500 und 0,4 g Tyzor^{®} TBT versetzt. Die Reaktionsmischung wird unter Schutzgas (Stickstoff) langsam innerhalb von 2 Stunden auf 190°C erwärmt und unter kontinuierlicher Wasserabscheidung im Vakuum bei sukzessiver Temperaturerhöhung auf 210°C 18 Stunden lang verestert. Nach Abkühlung auf etwa 100°C und Filtration wird das Produkt als Filtrat erhalten.
Das Produkt besitzt eine Säurezahl von 8 und eine Iodzahl von 2. Die Farbzahl nach Hazen liegt bei 100. Die Konsistenz ist (bei 20 °C) wachsartig-fest.

### Beispiel E

### Synthese eines Polyethylenglycol-600-polyhydroxystearats

836,5 g (2,72 mol) 12-Hydroxystearinsäure, 163,5 g (0,272 mol) Polyethylenglycol-600 und 0,1 g Zinnoxid werden unter Stickstoff stufenweise auf 240°C aufgeheizt. Nach Beendigung der Wasserabscheidung wird Vakuum angelegt und weiter kondensiert, bis keine weitere Absenkung der Säurezahl zu beobachten ist. Nach Abkühlung auf 100°C und Zugabe von 0,5% Filterhilfsmittel (Hyflow^{®} Supercel) wird das Produkt filtriert.
Das Produkt (Filtrat) besitzt eine Säurezahl von 15. Die Farbe ist dunkelbraun, die Farbzahl nach Hazen bzw. Gardner ist nicht bestimmbar. Die Konsistenz ist (bei 20°C) flüssig-fest.

### Beispiel F

### Synthese eines Polyethylenglycol-3000-polyhydroxystearats

711 g (2,32 mol) 12-Hydroxystearinsäure, 289 g (0,483 mol) Polyethylenglycol-3000 und 0,2 g Tetrabutyltitanat werden unter Stickstoff stufenweise auf 240°C aufgeheizt. Nach Beendigung der Wasserabscheidung wird Vakuum angelegt und weiter kondensiert, bis keine weitere Absenkung der Säurezahl zu beobachten ist. Nach Abkühlung auf 100°C und Zugabe von 0,5% Filterhilfsmittel (Hyflow^{®} Supercel) wird das Produkt filtriert.

Das Produkt (Filtrat) besitzt eine Säurezahl von 18. Die Farbe ist dunkelbraun, die Farbzahl nach Hazen bzw. Gardner ist nicht bestimmbar. Die Konsistenz ist (bei 20°C) wachsartig-fest.

### II. Herstellung der erfindungsgemäßen Emulgator-Konzentrate auf Basis der Polyethylenglycol-Fettsäure-Diester

Die Herstellung der flüssigen Polyethylenglycol-polyhydroxystearat-Mischungen erfolgte nach folgendem Verfahren: Das zur Compoundierung vorgesehene Öl (bzw. Ölmischung) wird im Mischgefäß vorgelegt, das aufgeschmolzene Polyethylenglycol-polyhydroxystearat zugegeben. Die Mischung wird - ggf. nach weiterer Erwärmung - homogenisiert und ggf. mit weiteren nichtwässrigen Additiven unter Rühren versetzt. Zuletzt wird das Wasser unter Rühren zugegeben.

### Beispiel 1

Nach oben beschriebenem Mischungs-Verfahren wird ein Compound, bestehend aus 55 g Di-n-Octylcarbonat und 41 g des Polyethylenglycol-Fettsäure-Diesters gemäß Beispiel A und 4 g deionisiertem Wasser hergestellt. Die Mischung ist bei 20 °C klar-flüssig (Viskosität Brookfield 20°C: ca. 2.000 mPa·s; Spindel 5 / 10 Umdrehungen pro Minute)

### Beispiel 2

Nach oben beschriebenem Mischungs-Verfahren wird eine Mischung, bestehend aus 40,5 g Di-n-Octylcarbonat und 55 g des Polyethylenglycol-Fettsäure-Diesters gemäß Beispiel A und 4,5 g deionisiertem Wasser hergestellt. Die Mischung ist bei 20 °C klar-flüssig (Viskosität Brookfield 20°C: 9.000 mPa s; Spindel 5/10 Umdrehungen pro Minute)

### Beispiel 3

Nach oben beschriebenem Mischungs-Verfahren wird eine Mischung, bestehend aus 19,5 g Di-n-Octylcarbonat und 77,5 g des Polyethylenglycol-Fettsäure-Diesters gemäß Beispiel A und 3 g deionisiertem Wasser hergestellt. Die Mischung ist bei 20 °C klar-flüssig.

### Beispiel 4

Nach oben beschriebenem Mischungs-Verfahren wird eine Mischung, bestehend aus 40,5 g Di-n-Octylether und 55 g des Polyethylenglycol-Fettsäure-Diesters gemäß Beispiel A und 4,5 g deionisiertem Wasser hergestellt. Die Mischung ist bei 20 °C klar-flüssig (Viskosität Brookfield 20°C: 8.500 mPa s; Spindel 5/10 Umdrehungen pro Minute)

### Beispiel 5

Nach oben beschriebenem Mischungs-Verfahren wird eine Mischung, bestehend aus 24 g Di-n-Octylether und 73 g des Polyethylenglycol-Fettsäure-Diesters gemäß Beispiel A und 3 g deionisiertem Wasser hergestellt. Die Mischung ist bei 20°C klar-flüssig.

### Beispiel 6

Nach oben beschriebenem Mischungs-Verfahren wird ein Compound, bestehend aus 55 g Di-n-Octylcarbonat und 41 g des Polyethylenglycol-Fettsäure-Diesters gemäß Beispiel D und 4 g deionisiertem Wasser hergestellt. Die Mischung ist bei 15°C klar-flüssig (Viskosität Brookfield 15°C: ca. 1.200 mPa s; Spindel 5/10 Umdrehungen pro Minute)

### Beispiel 7

Nach oben beschriebenem Mischungs-Verfahren wird ein Compound, bestehend aus 38 g Di-n-Octylcarbonat und 57 g des Polyethylenglycol-Fettsäure-Diesters gemäß Beispiel D und 5 g deionisiertem Wasser hergestellt. Die Mischung ist bei 20°C klar-flüssig (Viskosität Brookfield 20°C: ca. 9.800 mPa s; Spindel 5/10 Umdrehungen pro Minute)

### Beispiel 8

Nach oben beschriebenem Mischungs-Verfahren wird eine Mischung, bestehend aus 39 g Di-n-Octylcarbonat und 57 g des Polyethylenglycol-Fettsäure-Diesters gemäß Beispiel D und 4 g deionisiertem Wasser hergestellt. Die Mischung ist bei 20 °C klar-flüssig (Viskosität Brookfield 20°C: 5.900 mPa s; Spindel 5/10 Umdrehungen pro Minute)

### Beispiel 9

Nach oben beschriebenem Mischungs-Verfahren wird eine Mischung, bestehend aus 40,5 g Di-n-Octylcarbonat und 55,0 g des Polyethylenglycol-Fettsäure-Diesters gemäß Beispiel D und 4,5 g deionisiertem Wasser hergestellt. Die Mischung ist bei 20°C klar-flüssig (Viskosität Brookfield 20°C: 6.200 mPa·s; Spindel 5/10 Umdrehungen pro Minute)

### Beispiel 10

Nach oben beschriebenem Mischungs-Verfahren wird eine Mischung, bestehend aus 38 g Di-n-Octylether und 57 g des Polyethylenglycol-Fettsäure-Diesters gemäß Beispiel D und 5 g deionisiertem Wasser hergestellt. Die Mischung ist bei 20°C klar-flüssig.

### Beispiel 11

Nach oben beschriebenem Mischungs-Verfahren wird eine Mischung, bestehend aus 24 g Di-n-Octylether und 73 g des Polyethylenglycol-Fettsäure-Diesters gemäß Beispiel D und 3 g deionisiertem Wasser hergestellt. Die Mischung ist bei 20 °C klar-flüssig.

### Beispiel 12

Nach oben beschriebenem Mischungs-Verfahren wird eine Mischung, bestehend aus 47,5 g Myritol 331 (Cocoglycerides) und 47,5 g des Polyethylenglycol-Fettsäure-Diesters gemäß Beispiel D und 5 g deionisiertem Wasser hergestellt. Die Mischung ist bei 20 °C klar-flüssig.

### Beispiel 13

Nach oben beschriebenem Mischungs-Verfahren wird eine Mischung, bestehend aus 55 g Färberdistelöl und 41 g des Polyethylenglycol-Fettsäure-Diesters gemäß Beispiel D und 4 g deionisiertem Wasser hergestellt. Die Mischung ist bei 20°C klar-flüssig (Viskosität Brookfield 20°C: 9.000 mPa·s; Spindel 5 / 10 Umdrehungen pro Minute)

### Beispiel 14

Nach oben beschriebenem Mischungs-Verfahren wird eine Mischung, bestehend aus 55 g Eutanol G (Octyl-dodecanol) und 41 g des Polyethylenglycol-Fettsäure-Diesters gemäß Beispiel D und 4 g deionisiertem Wasser hergestellt. Die Mischung ist bei 20 °C klar-flüssig (Viskosität Brookfield 20°C: 9.000 mPa*s; Spindel 5/10 Umdrehungen pro Minute)

### Beispiel 15

Nach oben beschriebenem Mischungs-Verfahren wird eine Mischung, bestehend aus 55 g Paraffinöl dünnflüssig und 41 g des Polyethylenglycol-Fettsäure-Diesters gemäß Beispiel D und 4 g deionisiertem Wasser hergestellt. Die Mischung ist bei 20 °C klar-flüssig.

### Beispiel 16

Nach oben beschriebenem Mischungs-Verfahren wird eine Mischung, bestehend aus 47,5 g Di-n-Octylether und 47,5 g des Polyethylenglycol-Fettsäure-Diesters gemäß Beispiel E und 5 g deionisiertem Wasser hergestellt. Die Mischung ist bei 20 °C klar-flüssig.

### Beispiel 17

Nach oben beschriebenem Mischungs-Verfahren wird eine Mischung, bestehend aus 47,5 g Di-n-Octylether und 47,5 g des Polyethylenglycol-Fettsäure-Diesters gemäß Beispiel B und 5 g deionisiertem Wasser hergestellt. Die Mischung ist bei 20 °C klar-flüssig.

### Beispiel 18

Nach oben beschriebenem Mischungs-Verfahren wird eine Mischung, bestehend aus 47,5 g Di-n-Octylether und 47,5 g des Polyethylenglycol-Fettsäure-Diesters gemäß Beispiel C und 5 g deionisiertem Wasser hergestellt. Die Mischung ist bei 20 °C klar-flüssig.

### Beispiel 19

Nach oben beschriebenem Mischungs-Verfahren wird eine Mischung, bestehend aus 47,5 g Di-n-Octylether und 47,5 g des Polyethylenglycol-Fettsäure-Diesters gemäß Beispiel F und 5 g deionisiertem Wasser hergestellt. Die Mischung ist bei 20 °C klar-flüssig.

### Beispiel 20

Nach oben beschriebenem Mischungs-Verfahren wird eine Mischung, bestehend aus 46 g Di-n-Octylcarbonat, 46 g des Polyethylenglycol-Fettsäure-Diesters gemäß Beispiel C, 4 g Glycerin und 4 g deionisiertem Wasser hergestellt. Die Mischung ist bei 20 °C klar-flüssig (Viskosität Brookfield 20°C: ca. 3.000 mPa s; Spindel 5/10 Umdrehungen pro Minute)

### Beispiel 21

Nach oben beschriebenem Mischungs-Verfahren wird eine Mischung, bestehend aus 42 g Di-n-Octylcarbonat, 52 g des Polyethylenglycol-Fettsäure-Diesters gemäß Beispiel C, 2 g Butylenglycol (1,3-Butandiol) und 4 g deionisiertem Wasser hergestellt. Die Mischung ist bei 20 °C klar-flüssig (Viskosität Brookfield 20°C: ca. 7.000 mPa· s; Spindel 5/10 Umdrehungen pro Minute)

### Beispiel 22

Nach oben beschriebenem Mischungs-Verfahren wird eine Mischung, bestehend aus 50,5 g Di-n-Octylcarbonat, 41,5 g des Polyethylenglycol-Fettsäure-Diesters gemäß Beispiel C, 4 g Butylenglycol (1,3-Butandiol) und 4 g deionisiertem Wasser hergestellt. Die Mischung ist bei 20 °C klar-flüssig (Viskosität Brookfield 20°C: ca. 4.000 mPa·s; Spindel 5/10 Umdrehungen pro Minute)

### Beispiel 23

Nach oben beschriebenem Mischungs-Verfahren wird eine Mischung, bestehend aus 37 g Di-n-Octylcarbonat, 47 g des Polyethylenglycol-Fettsäure-Diesters gemäß Beispiel C, 10 g Eutanol G (Octyldodecanol) und 6 g deionisiertem Wasser hergestellt. Die Mischung ist bei 20 °C klar-flüssig (Viskosität Brookfield 20°C: ca. 7.000 mPa*s; Spindel 5/10 Umdrehungen pro Minute). Die Mischung ist bei 15 °C klar-flüssig (Viskosität Brookfield 15°C: ca. 9.000 mPa·s; Spindel 5 /10 Umdrehungen pro Minute).

### Beispiel 24

Nach oben beschriebenem Mischungs-Verfahren wird eine Mischung, bestehend aus 42 g Di-n-Octylcarbonat, 52 g des Polyethylenglycol-Fettsäure-Diesters gemäß Beispiel C, 2 g Butylenglycol (1,3-Butandiol) und 4 g deionisiertem Wasser hergestellt. Die Mischung ist bei 20 °C klar-flüssig (Viskosität Brookfield 20°C: ca. 7.000 mPa·s; Spindel 5/10 Umdrehungen pro Minute)

### Beispiel 25

Nach oben beschriebenem Mischungs-Verfahren wird eine Mischung, bestehend aus 40 g Di-n-Octylcarbonat, 20 g Cyclomethicon (Dow-Corning 245), 36 g des Polyethylenglycol-Fettsäure-Diesters gemäß Beispiel A und 4 g deionisiertem Wasser hergestellt. Die Mischung ist bei 20 °C klar-flüssig (Viskosität Brookfield 20°C: ca.1.000 mPa·s; Spindel 5 / 10 Umdrehungen pro Minute).

### Beispiel 26

Nach oben beschriebenem Mischungs-Verfahren wird eine Mischung, bestehend aus 11 g Eutanol G (Octyldodecanol), 85 g des Polyethylenglycol-Fettsäure-Diesters gemäß Beispiel D und 4 g deionisiertem Wasser hergestellt. Die Mischung ist bei 20 °C klar-flüssig (Viskosität Brookfield 20°C: ca. 17.000 mPa s; Spindel 5 / 10 Umdrehungen pro Minute)

### Beispiel 27

Nach oben beschriebenem Mischungs-Verfahren wird eine Mischung, bestehend aus 8 g Eutanol G 16 (Hexyldecanol), 88 g des Polyethylenglycol-Fettsäure-Diesters gemäß Beispiel A und 4 g deionisiertem Wasser hergestellt. Die Mischung ist bei 20 °C klar-flüssig.

### III. Vergleichsbeispiele

### Vergleichsbeispiel 1

Nach oben beschriebenem Mischungs-Verfahren wird eine Mischung, bestehend aus 45 g Di-n-Octylcarbonat und 45 g des Polyethylenglycol-Fettsäure-Diesters gemäß Beispiel A und 10 g deionisiertem Wasser hergestellt. Die Mischung ist bei 20 °C vergelt (Viskosität Brookfield 20°C: nicht messbar).

### Vergleichsbeispiel 2

Nach oben beschriebenem Mischungs-Verfahren wird eine Mischung, bestehend aus 50 g Di-n-Octylcarbonat und 50 g des Polyethylenglycol-Fettsäure-Diesters gemäß Beispiel A (ohne Wasserzusatz) hergestellt. Die Mischung ist bei 20 °C wachsartig-fest. (Viskosität Brookfield 20°C: nicht messbar).

### Vergleichsbeispiel 3

Nach oben beschriebenem Mischungs-Verfahren wird eine Mischung, bestehend aus 35 g Di-n-Octylether und 55 g des Polyethylenglycol-Fettsäure-Diesters gemäß Beispiel A und 10 g deionisiertem Wasser hergestellt. Die Mischung ist bei 20 °C vergelt (Viskosität Brookfield 20°C: nicht messbar).

### Vergleichsbeispiel 4

Nach oben beschriebenem Mischungs-Verfahren wird eine Mischung, bestehend aus 50 g Di-n-Octylether und 50 g des Polyethylenglycol-Fettsäure-Diesters gemäß Beispiel A (ohne Wasserzusatz) hergestellt. Die Mischung ist bei 20 °C wachsartig-fest. (Viskosität Brookfield 20°C: nicht messbar).

### Vergleichsbeispiel 5

Nach oben beschriebenem Mischungs-Verfahren wird eine Mischung, bestehend aus 46 g Di-n-Octylcarbonat, 46 g des Polyethylenglycol-Fettsäure-Diesters gemäß Beispiel C und 4 g Glycerin (ohne Wasserzusatz) hergestellt. Die Mischung ist bei 20 °C wachsartig-fest. (Viskosität Brookfield 20°C: nicht messbar).

### Vergleichsbeispiel 6

Nach oben beschriebenem Mischungs-Verfahren wird eine Mischung, bestehend aus 42 g Di-n-Octylcarbonat, 52 g des Polyethylenglycol-Fettsäure-Diesters gemäß Beispiel C und 4 g Butylenglycol (1,3-Butandiol) ohne Wasserzusatz hergestellt. Die Mischung ist bei 20 °C wachsartig-fest. (Viskosität Brookfield 20°C: nicht messbar).

### Vergleichsbeispiel 7

Nach oben beschriebenem Mischungs-Verfahren wird eine Mischung, bestehend aus 40 g Di-n-Octylcarbonat und 10 g Eutanol G (Octyldodecanol), 50 g des Polyethylenglycol-Fettsäure-Diesters gemäß Beispiel C (ohne Wasserzusatz) hergestellt. Die Mischung ist bei 20 °C wachsartig-fest. (Viskosität Brookfield 20°C: nicht messbar).

### Formulierungsbeispiele

In den Tabellen 1 und 2 sind kosmetische Zusammensetzungen offenbart, die mit Hilfe der Emulgator-Konzentrate formuliert wurden. Die Mengen der einzelnen Komponenten sind in Gew.-% der handelsüblichen Substanz bezogen auf die Gesamtzusammensetzung angegeben.

Unter Verwendung ausgewählter Emulgator-Konzentrate wurden verschiedene kosmetische Zusammensetzungen gemäß den allgemein üblichen und bekannten Methoden über Heiß-Verfahren ("H") oder Kalt-Verfahren ("C") hergestellt.

Die Viskositätsmessungen wurden mit einem Brookfield RVF Viskosimeter bei 23°C durchgeführt (je nach Viskosität: Spindel 5, 10 Umdrehungen pro Minute, 23°C; bzw. Spindel TE, 4 Umdrehungen pro Minute, Helipath).

Die Bewertung der Struktur und der Stabilität der Formulierungen wurden von einer geschulten Testperson durchgeführt. Die Kriterien "Struktur" und "Stabilität" wurden auf einer Skala von - 2 bis + 2 bewertet: Struktur (-2: sehr inhomogen bis +2: sehr homogen) und Stabilität (-2: gering bis +2: hoch).

**Tabelle 1: Kosmetische Emulsionen**

| Bestandteile/ (INCI) | Beispiel 1 H | Beispiel 2 H | Beispiel 3 H | Beispiel 4 C | Beispiel 5 H | Beispiel 6 C | Beispiel 7 H |
|---|---|---|---|---|---|---|---|
| **Emulgator-Konzentrat gemäß Beispiel 2** | | | | | | | |
| **Emulgator-Konzentrat gemäß Beispiel 6** | | | | | | | |
| **Emulgator-Konzentrat gemäß Beispiel 9** | 8,33 | 3,33 | 3,33 | 3,33 | 3,33 | 3,33 | 5,0 |
| Lameform® TGI (Polyglyceryl-3 Diisostearat) | | 2,0 | | | | | |
| Dehymuls® PGPH (Polyglyceryl-2 Dipolyhydroxystearat) | | | 2,0 | 2,0 | 2,0 | 2,0 | |
| Cetiol® CC (Dicaprylyl Carbonate) | | 6,67 | 4,67 | 4,67 | 3,17 | 3,17 | 4,0 |
| Cetiol® OE (Dicaprylyl Ether) | 2,67 | | | | 4,5 | 4,5 | |
| Cetiol® A (Hexyl Laurat) | 6,0 | | 6,0 | 6,0 | | | 6,0 |
| Cetiol® SN (Cetearyl Isononanoat) | 10,0 | | 7,0 | 7,0 | 4,8 | 4,8 | 10,0 |
| Eutanol® G16 (Hexyldecanol) | 3,0 | | 3,0 | 3,0 | 2,2 | 2,2 | 3,0 |
| Myritol® 331 (Cocoglycerides) | | | | | | | |
| Cetiol® OE (Dicaprylyl Ether) | | | | | | | |
| Cetiol® PGL (Hexyldecanol und Hexyldecyl Laurat) | | | | | | | |
| Cetiol® 868 (Octyl Stearat) | | | | | | | |
| Isopropylmyristat | | 8,0 | | | | | |
| Vaseline | | 2,0 | | | | | |
| Zincum® N29 (Zink Stearat) | | 1,0 | | | | | |
| Rezal® 36GP (Aluminium Zirconium Tetrachlorohydrex GLycin) | | | | | 40,0 | 40,0 | |
| Copherol® F1300 (Tocopherol) | | | | | | | |
| Butylenglycol 1,3 | | 3,0 | | | | | |
| MgSO₄ 7H₂O Magnesium Sulfat | 1,0 | 1,0 | 1,0 | 1,0 | 1,0 | 1,0 | 1,0 |
| Glycerin 99,5%ig | 3,0 | 2,0 | 3,0 | 3,0 | 3,0 | 3,0 | 3,0 |
| Wasser, deionisiert Konservierungsmittel | ad 100 | ad 100 | ad 100 | ad 100 | ad 100 | ad 100 | ad 100 |
| | | | | | | | |
| **Bewertung** | | | | | | | |
| Struktur der Emulsion | +2 | +2 | +2 | +2 | +2 | +2 | +2 |
| Viskosität der Emulsion | 1200 | 13600 | 5200 | 11200 | 6800 | 20000 | 1600 |
| Stabilitäten bei RT/- 5°C/40°C/45°C/50°C (über 12 Wochen) | +2 | +2 | +2 | +2 | +2 | +2 | +2 |

| | | | | | | | |
|---|---|---|---|---|---|---|---|
| Die Stabilitäten wurden bei allen vier Temperaturen mit +2 bewertet. H: Herstellung Heiß-Verfahren C: Herstellung-Kaft-Verfahren | | | | | | | |

**Tabelle 2:**

| Bestandteile/ (INCI) | Beispiel 8 H | Beispiel 9 H | Beispiel 10 H | Beispiel 11 C | Beispiel 12 H | Beispiel 13 C | Beispiel 14 H | Beispiel 15 H | Beispiel 16 H |
|---|---|---|---|---|---|---|---|---|---|
| **Emulgator-Konzentrat gemäß Beispiel 2** | | | 3,33 | 3,33 | 3,33 | 3,33 | | | |
| **Emulgator-Konzentrat gemäß Beispiel 6** | | | | | | | 7,2 | 7,2 | 7,2 |
| **Emulgator-Konzentrat gemäß Beispiel 9** | 7,5 | 3,5 | | | | | | | |
| Lameform® TGI (Polyglyceryl-3 Diisostearat) | | 2,0 | | | | | | | |
| Dehymuls® PGPH (Polyglyceryl-2 Dipolyhydroxystearat) | | | 2,0 | 2,0 | 2,0 | 2,0 | | | |
| Cetiol® CC (Dicaprylyl Carbonate) | 2,0 | 6,5 | 4,67 | 4,67 | 3,17 | 3,17 | 1,8 | | 1,8 |
| Cetiol® OE (Dicaprylyl Ether) | | | | | 4,5 | 4,5 | | 1,8 | |
| Cetiol® A (Hexyl Laurat) | | | 6,0 | 6,0 | | | 6,0 | 6,0 | 6,0 |
| Cetiol® SN (Cetearyl Isononanoat) | | | 7,0 | 7,0 | 4,8 | 4,8 | 10,0 | | |
| Eutanol® G16 (Hexyldecanol) | | | 3,0 | 3,0 | 2,2 | 2,2 | 3,0 | 3,0 | 3,0 |
| Myritol® 331 (Cocoglycerides) | 6,0 | | | | | | | | |
| Cetiol® OE (Dicaprylyl Ether) | | | | | | | | | |
| Cetiol® PGL (Hexyldecanol und Hexyldecyl Laurat) | 5,0 | | | | | | | | |
| Cetiol® 868 (Octyl Stearat) | | | | | | | | 10,0 | 10,0 |
| Isopropylmyristat | | 8,0 | | | | | | | |
| Vaseline | | 2,0 | | | | | | | |
| Zincum® N29 (Zink Stearat) | 1,0 | 1,0 | | | | | | | |
| Rezal® 36GP (Aluminium Zirconium Tetrachlorohydrex GLY) | | | | | 40,0 | 40,0 | | | |
| Copherol® F1300 (Tocopherol) | 1,0 | 0,0 | | | | | | | |
| Butylenglycol 1,3 | | 3,0 | | | | | | | |
| MgSO₄·7H₂O Magnesium Sulfat | 0,5 | 1,0 | 1,0 | 1,0 | 1,0 | 1,0 | 1,0 | 1,0 | 1,0 |
| Glycerin 99,5%ig | 5,0 | 2,0 | 5,0 | 5,0 | 5,0 | 5,0 | 5,0 | 5,0 | 5,0 |
| Wasser, deion. Konservierungsmittel | ad 100 | ad 100 | ad 100 | ad 100 | ad 100 | ad 100 | ad 100 | ad 100 | ad 100 |
| | | | | | | | | | |
| **Bewertung** | | | | | | | | | |
| Struktur der Emulsion | +2 | +2 | +2 | +2 | +2 | +2 | +2 | +2 | +2 |
| Viskosität der Emulsion | *125000 | 16800 | 9600 | 7600 | 8800 | 24800 | 1600 | 1200 | 1200 |
| Stabilitäten bei RT/-5°C/40°C/45°C/50°C (über 12 Wochen) | +2 | +2 | +2 | +2 | +2 | +2 | +2 | +2 | +2 |

| | | | | | | | | | |
|---|---|---|---|---|---|---|---|---|---|
| *Brookfield RVF, TE-Spindel mit 4 Umdrehungen/Minute, + 23°C Die Stabilitäten wurden bei allen vier Temperaturen mit +2 bewertet. H: Herstellung Heiß-Verfahren C: Herstellung Kalt-Verfahren | | | | | | | | | |

### Anhang

1) Hyflow^{®} Supercel
   INCI: Kieselgur
   Hersteller: Manville Corporation
2) Tyzor^{®} TPT
   INCI: Tetraisopropyltitanat
   Hersteller: E.I. du Pont de Nemours and Company

## Patentansprüche

1. Bei 20°C fließfähiges Emulgator-Konzentrat enthaltend
(a) bis zu 90 Gew.-% eines Polyethylenglykol Fettsäure-Diesters auf Basis einer C16-C22-Fettsäure, einer C16-C22-Hydroxyfettsäure, Polyhydroxystearinsäure oder Poly-(C16-C22-Hydroxyfettsäure)
(b) bis zu 80 Gew.-% eines bei 25°C flüssigen Ölkörpers oder Ölkörpergemisches und
(c) 2 - 8 Gew.-% Wasser.

2. Emulgator-Konzentrat gemäß Anspruch 1, **dadurch gekennzeichnet, daß** es keine weiteren nichtionischen, anionischen, kationischen amphoteren und/oder zwitterionischen oberflächenaktiven Substanzen enthält.

3. Emulgator-Konzentrat gemäß wenigstens einem der Ansprüche 1 bis 2, **dadurch gekennzeichnet, daß** die Ölkörper ausgewählt sind aus der Gruppe der Dialkylether, der Dialkylcarbonate, der Triglyceride, der Ester, der Kohlenwasserstoffe, der verzweigten C12-C24-Fettalkohole oder einem beliebigen Gemisch dieser Substanzen.

4. Emulgator-Konzentrat gemäß wenigstens einem der Ansprüche 1 bis 3, **dadurch gekennzeichnet, daß** es bei 20°C eine Viskosität von weniger als 20.000 mPa·s aufweist, gemessen mit einem Brookfield-Viskosimeter mit Spindel 5 bei 10 Umdrehungen pro Minute.

5. Emulgator-Konzentrat gemäß wenigstens einem der Ansprüche 1 bis 4, **dadurch gekennzeichnet, daß** es transparent ist.

6. Emulgator-Konzentrat gemäß wenigstens einem der Ansprüche 1 bis 5, **dadurch gekennzeichnet, dass** es ein Hydrotrop ausgewählt aus der Gruppe der Polyole 2 bis 15 Kohlenstoffatomen und mindestens 2 Hydroxylgruppen enthält.

7. Emulgator-Konzentrat gemäß einem der Ansprüche 1 bis 6, **dadurch gekennzeichnet, daß** es als Komponente a) einen Ester enthält, der **dadurch** erhältlich ist, dass man eine Fettsäure und/oder Hydroxyfettsäure mit 16 bis 22 C-Atomen oder eine entsprechende Polyfettsäure und/oder Poly(C16-C22-Hydroxyfettsäure) mit einem Eigenkondensationsgrad von 2 bis 20, insbesondere 2 bis 10, mit einem Polyethylenglykol unter Durchführung einer Veresterungsreaktion in Gegenwart eines Katalysators umsetzt, der eine anorganische Phosphor(I)-Verbindung sowie ein Titanat umfasst, wobei die
a) die Carbonylverbindung und die anorganische Phosphor(I)-Verbindung miteinander vermischt werden,
b) die erhaltene Mischung filtriert wird und anschließend
c) der filtrierten Mischung der Alkohol und das Titanat zugesetzt werden und die Veresterungsreaktion durchgeführt wird.

8. Verfahren zur Herstellung von Emulsionen, **dadurch gekennzeichnet, daß** eine Emulgator-Konzentrat gemäß einem der Ansprüche 1 bis 7 mit einer Öl - und einer Wasserphase nach einem Heiß- oder Kaltprozess emulgiert wird.

9. Verwendung der Emulgator-Konzentrate gemäß einem der Ansprüche 1 bis 7 in kosmetischen Emulsionen.

10. Kosmetisches Mittel enthaltend 1 - 30 Gew.-% eines Emulgator-Konzentrates gemäß wenigstens einem der Ansprüche 1 bis 7.

## Claims

1. An emulsifier concentrate flowable at 20°C containing
(a) up to 90% by weight of a polyethylene glycol fatty acid diester based on a C₁₆₋₂₂ fatty acid, a C₁₆₋₂₂ hydroxyfatty acid, polyhydroxystearic acid or poly-(C₁₆₋₂₂ hydroxyfatty acid),
(b) up to 80% by weight of an oil component liquid at 25°C or a mixture of such oil components and
(c) 2 to 8% by weight water.

2. An emulsifier concentrate as claimed in claim 1, **characterized in that** it does not contain any other nonionic, anionic, cationic, amphoteric and/or zwitterionic surfactants.

3. An emulsifier concentrate as claimed in at least one of claims 1 to 2, **characterized in that** the oil components are selected from the group of dialkyl ethers, dialkyl carbonates, triglycerides, esters, hydrocarbons, branched C₁₂₋₂₄ fatty alcohols or mixtures thereof.

4. An emulsifier concentrate as claimed in at least one of claims 1 to 3, **characterized in that** it has a viscosity at 20°C of less than 20,000 mPa.s, as measured with a Brookfield viscosimeter, spindle 5, at 5 to 10 r.p.m.

5. An emulsifier concentrate as claimed in at least one of claims 1 to 4, **characterized in that** it is transparent.

6. An emulsifier concentrate as claimed in at least one of claims 1 to 5, **characterized in that** it contains a hydrotrope selected from the group of polyols containing 2 to 15 carbon atoms and at least 2 hydroxyl groups.

7. An emulsifier concentrate as claimed in any of claims 1 to 6, **characterized in that** it contains as component a) an ester obtainable by esterification of a fatty acid and/or hydroxyfatty acid containing 16 to 22 carbon atoms or a corresponding polyfatty acid and/or poly-(C₁₆₋₂₂-hydroxyfatty acid) having a degree of selfcondensation of 2 to 20 and more particularly 2 to 10 with a polyethylene glycol in the presence of a catalyst comprising an inorganic phosphorus(I) compound and a titanate,
a) the carbonyl compound and the inorganic phosphorus(I) compound being mixed with one another,
b) the mixture obtained being filtered and
c) the alcohol and the titanate being added to the filtered mixture and the esterification being carried out.

8. A process for the production of emulsions, **characterized in that** the emulsifier concentrate claimed in any of claims 1 to 7 is emulsified with an oil phase and a water phase by a hot or cold process.

9. The use of the emulsifier concentrates claimed in any of claims 1 to 7 in cosmetic emulsions.

10. A cosmetic preparation containing 1 to 30% by weight of the emulsifier concentrate claimed in at least one of claims 1 to 7.

## Revendications

1. Concentré d'émulsifiant fluide à 20 °C contenant :
(a) jusqu'à 90 % en poids d'un diester d'acide gras et de polyéthylèneglycol à base d'un acide gras en C16-C22, d'un acide hydroxyaliphatique en C16-C22, d'un acide polyhydroxystéarique ou d'un poly(acide hydroxy aliphatique en C16-C22)
(b) jusqu'à 80 % en poids d'un corps huileux ou d'un mélange de corps huileux liquide à 25 °C et
(c) 2 % à 8 % en poids d'eau.

2. Concentré d'émulsifiant selon la revendication 1,
**caractérisé en ce qu'**
il ne contient pas d'autres substances tensioactives non ioniques, anioniques, cationiques, amphotères et/ou hermaphrodites.

3. Concentré d'émulsifiant selon l'une des revendications 1 à 2,
**caractérisé en ce que**
les corps huileux sont choisis dans le groupe constitué des éthers de dialkyles, des carbonates de dialkyles, des triglycérides, des esters, des hydrocarbures, des alcools gras en C12-C24 ramifiés ou d'un mélange quelconque de ces substances.

4. Concentré d'émulsifiant selon au moins l'une des revendications 1 à 3,
**caractérisé en ce qu'**
il présente à 20 °C une viscosité inférieure à 20 000 mPas, mesurée avec un viscosimètre Brookfield avec tige n° 5 à 10 tours par minute.

5. Concentré d'émulsifiant selon au moins l'une des revendications 1 à 4,
**caractérisé en ce qu'**
il est transparent.

6. Concentré d'émulsifiant selon l'une des revendications 1 à 5,
**caractérisé en ce qu'**
il contient un hydrotrope choisi dans le groupe des polyols comportant 2 à 15 atomes de carbone et au moins 2 groupes hydroxyle.

7. Concentré d'émulsifiant selon une des revendications 1 à 6,
**caractérisé en ce qu'**
il contient, comme composant a) un ester qui peut être obtenu en faisant réagir un acide gras et/ou un acide hydroxy aliphatique comportant 16 à 22 atomes de carbone ou un acide polyaliphatique correspondant et/ou un poly (acide hydroxy aliphatique en C16-C22) présentant un degré de condensation propre de 2 à 20, en particulier 2 à 10, avec un polyéthylèneglycol, en effectuant une réaction d'estérification en présence d'un catalyseur, qui comprend un composé du phosphore (I) inorganique ainsi qu'un titanate, sachant que la procédure suivie est la suivante :
(a) on mélange ensemble le composé carbonyle et le composé du phosphore(I) inorganique,
(b) on filtre le mélange obtenu et ensuite
(c) on ajoute l'alcool et le titanate au mélange filtré et on effectue la réaction d'estérification.

8. Procédé pour la préparation d'émulsions,
**caractérisé en ce qu'**
on émulsifie un concentré d'émulsifiant selon une des revendications 1 à 7 avec une phase huile et une phase eau après un procédé à chaud ou à froid.

9. Utilisation du concentré d'émulsifiant selon l'une des revendications 1 à 7 dans des émulsions cosmétiques.

10. Produit cosmétique contenant 1 %-30 % en poids d'un concentré d'émulsifiant selon au moins l'une des revendications 1 à 7.
